Europäisches Patentamt

European Patent Office

Office européen des brevets

(11) Publication number: **0 107 170**
A1

# EUROPEAN PATENT APPLICATION

(21) Application number: 83110379.1

(22) Date of filing: 18.10.83

(51) Int. Cl.³: **C 12 N 15/00**
// C12R1/85, C12R1/19

(30) Priority: 19.10.82 JP 182197/82

(43) Date of publication of application: 02.05.84
Bulletin 84/18

(84) Designated Contracting States: DE FR GB

(71) Applicant: Yamamoto, Masayuki, 1-24-11 Kamitsuruma Sagamihara-shi, Kanagawa-ken (JP)

(72) Inventor: Yamamoto, Masayuki, 1-24-11 Kamitsuruma Sagamihara-shi, Kanagawa-ken (JP)

(74) Representative: Vossius Vossius Tauchner Heunemann Rauh, Siebertstrasse 4 P.O. Box 86 07 67, D-8000 München 86 (DE)

(54) Novel plasmid vector.

(57) Disclosed is a plasmid vector which is a chimera plasmid constructed with a chromosomal DNA fragment of a microorganism belonging to the genus Schizosaccharomyces and a plasmid of a microorganism belonging to the genus Escherichia and is autonomously replicable in both microorganisms belonging to the genera Schizosaccharomyces and Escherichia.

pFYM 225 G4

VOSSIUS · VOSSIUS · TAUCHNER · HEUNEMANN · RAUH

PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

Our Ref.: S 677 EP
Case 333-EPC

Masayuki YAMAMOTO

Kanagawa-ken, Japan

TITLE OF THE INVENTION

NOVEL PLASMID VECTOR

## Background of the Invention

The present invention relates to a novel plasmid vector. More specifically, the present invention relates to a plasmid vector which is a chimera plasmid constructed with a chromosomal DNA fragment of a microorganism belonging to the genus Schizosaccharomyces and a plasmid of a microorganism belonging to the genus Escherichia and is autonomously replicable in both microorganisms belonging to the genera Schizosaccharomyces and Escherichia.

Host-vector systems for yeast have been developed mainly with baker's yeast Saccharomyces cerevisiae as known with YRp7, YEp13, YIp1, pJDB219 or the like [Akio Toh-e et al.: Protein, Nucleic Acid and Enzyme 26, 476, (1981)].

The study of Beach et al [D. Beach et al: Nature 290, 140 - 142 (1981)] relates to the host-vector system with Schizomycetes such as Schizosaccharomyces. Beach et al found that a shuttle vector pJDB248 containing the leucin-2 gene of Saccharomyces cerevisiae, 2 μm plasmid and plasmid pMB9 of Escherichia coli [J.D. Beggs et al.: Nature 275 104 - 109 (1978)] and its derivatives are applicable to the effective transformation of Schizosaccharomyces pombe.

The present inventor has cloned Schizosaccharomyces pombe DNA fragment which is able to complement PyrB (pyrimidine B) mutation of Escherichia coli into pBR322. The clones were reported as pFYM1, pFYM2 and pFYM3 [Yamamoto et al.: Mol. Gen. Genet. 182, 426 - 429 (1981)].

- 2 -

The present inventor has studied and found that a derivative of pFYM201 is autonomously replicable in both Escherichia coli and Schizosaccharomyces pombe and is applicable as plasmid vector in host-vector system for the genus Schizosaccharomyces.

## Summary of the Invention

The present invention provides a plasmid vector which is a chimera plasmid constructed with a chromosomal DNA fragment of a microorganism belonging to the genus Schizosaccharomyces and a plasmid of a microorganism belonging to the genus Escherichia and is autonomously replicable in both microorganisms belonging to the genera Schizosaccharomyces and Escherichia.

As the microorganism belonging to the genus Schizosaccharomyces, those belonging to the species Schizosaccharomyces pombe are employed.

As the chromosomal DNA fragment of a microorganism belonging to the genus Schizosaccharomyces, a DNA fragment which is able to complement ural mutation of Schizosaccharomyces pombe i.e. a DNA fragment containing the structural gene of aspartate transcarbamylase is employed. The DNA fragment of Schizosaccharomyces pombe can be prepared according to the method of Cryer et al [Method in Cell Biology 12, 39 (1975), Academic Press, New York, Prescott D.M. (ed)].

As the plasmids of a microorganism belonging to the genus Escherichia, the plasmids derived from Escherichia coli such as pBR322, pBR325, pBR327, pBR328, pMB9, pCR1, RSF2124, pBR313, pACYC177 and pACYC184 are used. These plasmids are prepared by the methods described in P. Guerry et al., J. Bacteriol. 116, 1064 (1973).

## Brief Description of the Drawings

Fig. 1 shows the structures of plasmids pFYM2, pFYM201 and pFYM225.

Fig. 2 shows the structure of plasmid pFYM225G4.

In the drawings, the thick line represents the DNA derived from chromosome of _Schizosaccharomyces pombe_, the fine line represents the DNA derived from pBR322, and the boxed line represents the DNA derived from transposon Tn903 existing on plasmid pAJ-43, and coding for an enzyme inactivating G418. The abbreviations represent the following restriction enzymes, respectively.

Ba: BamHI, Bg: BglII, C : ClaI, E.: EcoRI,

H : HindIII, Ps: PstI, Pv: PvuI, X.: XhoI.

## Detailed Description of the Invention

Preparation of the chimera plasmid is carried out by conventional recombinant DNA technology. For example, a plasmid DNA is digested with restriction enzymes and the enzyme is inactivated by heating. A DNA fragment derived from the genus _Schizosaccharomyces_ is ligated with the digested plasmid DNA according to the method of Williams _et al._, [B.G. Williams _et al._, J. Biol. Chem. _252_, 7344 - 7354 (1977)]. Selection of the plasmid of the present invention is carried out using a selection marker retained in the plasmid DNA.

Transformation of microorganisms of the genus _Escherichia_ such as _Escherichia coli_ with the plasmid vector of the present invention is carried out according to the method of Cohen _et al._ [S.N. Cohen: Proc. Natl. Acad. Sci. U.S.A. _69_, 2110 - 2114 (1972)]. Transformation of microorganisms of the genus _Schizosaccharomyces_ such as _Schizosaccharomyces pombe_ with the plasmid vector of the present invention is carried out according to the method of Beach _et al._ [D. Beach _et al._, Nature _290_, 140 - 142 (1981)].

The plasmid vector of the present invention is autonomously replicable in both <u>Escherichia</u> <u>coli</u> and <u>Schizosaccharomyces</u> <u>pombe</u> and is useful as a shuttle vector. Further, plural plasmid vectors of the present invention exist in a form of a polymer chain in a host microorganism. This is one of the characteristics which distinguish the present vector from known vectors of yeast.

The simultaneous use of the plasmid vector of the present invention and other plasmids such as pFYM2 in transformation treatment can elevate the ratio of transformation frequency.

Examples of the present invention are as follows.

Example 1

Construction of plasmid pFYM201:

In this example, 5 μg of plasmid pFYM2 [Mol. Gen. Genet. 182, 426 - 429 (1981)] DNA was dissolved in 50 μl of a mixture of 10 mM Tris-HCl (pH 7.5), 7 mM $MgCl_2$, 50 mM NaCl and 6 mM 2-mercaptoethanol. 10 units of restriction enzyme PvuII (product of Takara Shuzo Co.) was added and the mixture was incubated at 37°C for 2 hours. The reaction solution was heated at 65°C for 5 minutes to inactivate the enzyme and 5 μl of 3M sodium acetate and 125 μl

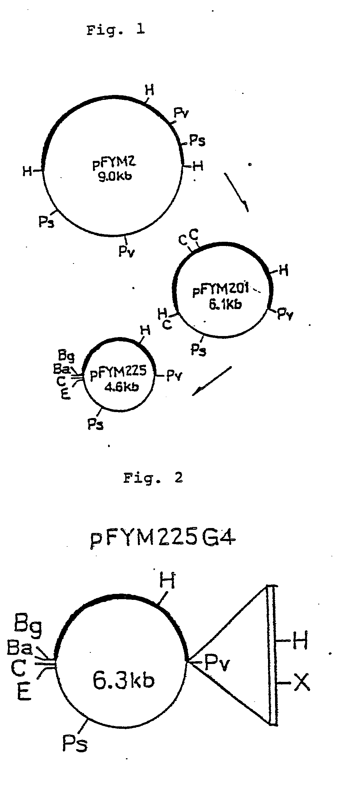

of ethanol were added. The mixture was allowed to stand at -20°C for 2 hours and subjected to centrifugation at 12,000 r.p.m. for 5 minutes to recover the DNA as precipitates. The whole DNA recovered was dissolved in 30 μl of a mixture of 20 mM Tris-HCl (pH 7.5), 10 mM MgCl$_2$, 10 mM dithiothreitol and 0.5 mM ATP and 4 units of T4DNA ligase (product of New England Biolabs, the same as hereinbelow) was added. The mixture was allowed to react at 4°C overnight. Escherichia coli PyrB mutant NF305 (PyrB43, leuB, hisG, lysA, argG, metB, recA, F$^-$) [Mol. Gen. Genet. 182, 426 - 429 (1981)] was transformed using the reaction solution according to a conventional method [S.N. Cohen: Proc. Natl. Acad. Sci., 69, 2110 - 2114 (1972)] and a transformant resistant to 40 μg/ml ampicillin (Ap$^R$) was selected from the obtained transformants. The transformant was cultured in an aqueous medium in a conventional way to isolate 50 μg of plasmid from the cultured medium. The plasmid thus obtained has the structure illustrated as plasmid pFYM201 in Fig. 1. The structure of pFYM201 was confirmed by agarose gel electrophoresis after digestion with HindIII, ClaI, PvuII and PstI alone or in combination. The size of plasmid pFYM201 is about 6.1 kilobases (Kb). Escherichia coli U-201 containing plasmid pFYM201 has been deposited with the Fermentation Research Institute, Agency of Industrial Science and Technology under accession number FERM P-6768 (FERM BP-348).

Example 2

Construction of plasmid pFYM225:

In this example, 3 μg of plasmid pFYM201 obtained in Example 1 was dissolved in 30 μl of a mixture of 10 mM Tris-HCl (pH 7.5), 7 mM MgCl$_2$ and 6 mM 2-mercaptoethanol. 6 units of restriction enzyme ClaI (product of Takara Shuzo Co.) was added and the mixture was incubated at 37°C for 2 hours. The reaction solution was heated at 65°C for 5 minutes to inactivate the enzyme and 3 μl of 3M sodium acetate and 75 μl of ethanol were added. The mixture was allowed to stand at -20°C for 2 hours and subjected to centrifugation at 12,000 r.p.m. for 5 minutes to recover the DNA as precipitates. The whole DNA recovered was dissolved in 30 µl of a solution containing 20 mM Tris-HCl (pH 7.5), 10 mM $MgCl_2$, 10 mM dithiothreitol and 0.5 mM ATP. 4 units of T4DNA ligase was added and the mixture was allowed to react at 4°C overnight. Escherichia coli PyrB mutant NF305 was transformed using the reaction solution as in Example 1 and a transformant resistant to 40 µg/ml ampicillin was obtained. The transformant was cultured in an aqueous medium in a conventional way and 30 µg of plasmid was isolated from the culture medium by the same method as in Example 1. The plasmid thus obtained has the structure illustrated as pladmid pFYM225 in Fig. 1. The structure of pFYM225 was confirmed by agarose gel electrophoresis after digestion with EcoRI, ClaI, BamHI, BglII, HindIII, PvuII and PstI alone or in combination. The size of plasmid pFYM225 is about 4.6 Kb.

Escherichia coli U-225 containing plasmid pFYM225 has been deposited with the Fermentation Research Institute, Agency of Industrial Science and Technology under accession number FERM P-6769 (FERM BP-349).

Example 3

Construction of plasmid pFYM225G4:

In this example, 2 µg of plasmid pFYM225 obtained in Example 2 was dissolved in 30 µl of a mixture of 10 mM Tris-HCl (pH 7.5), 7 mM $MgCl_2$, 50 mM NaCl and 6 mM 2-mercaptoethanol. 4 units of restriction enzyme PvuII (product of Takara Shuzo Co., the same as hereinbelow) was added and the mixture was incubated at 37°C for 2 hours. The reaction solution was heated at 65°C for 5 minutes to inactivate the enzyme.

Separately, 5 µg of plasmid pAO43 [A. Oka et al. Nature 276, 845 - 847 (1978); this plasmid includes a DNA-sequence coding for an enzyme which inactivates the antibiotic gentamycin G-418] was dissolved in 50 µl of a mixture of 10 mM Tris-HCl (pH 7.5), 7 mM $MgCl_2$, 50 mM NaCl and 6 mM 2-mercaptoethanol. 10 units of PvuII was added and the mixture was incubated at 37°C for 3 hours. The reaction solution was heated at 65°C for 5 minutes to inactivate the enzyme.

Each reaction solution was treated with ethanol to obtain the DNA as precipitates in the same way as in Example 2. The whole of each DNA was dissolved in 30 µl of a solution containing 20 mM Tris-HCl (pH 7.5), 10 mM MgCl$_2$ and 10 mM dithiothreitol. 15 µl each of the solutions were mixed and 0.5 mM (final concentration) ATP and 4 units (final amount) of T4DNA ligase were added. The mixture was allowed to react at 4°C overnight.

Escherichia coli PyrB mutant NF305 was transformed using the reaction mixture as in Example 1 and a transformant resistant to 40 µg/ml ampicillin and 50 µg/ml kanamycin was obtained. The transformant was cultured in an aqueous medium in a conventional way and 55 µg of plasmid was isolated from the cultured medium as in Example 1. The plasmid has the structure illustrated as plasmid pFYM225G4 in Fig. 2. The structure of plasmid pFYM225G4 was confirmed by agarose gel electrophoresis after digestion with PstI, EcoRI, ClaI, BamHI, BglII, HindIII, PvuII and XhoI alone or in combination. The size of plasmid pFYM225G4 is about 6.3 Kb.

Escherichia coli U-225G4 containing plasmid pFYM225G4 has been deposited with the Fermentation Research Institute, Agency of Industrial and Science Technology under accession number FERM P-6770 (FERM BP-350).

Example 4

Schizosaccharomyces pombe JY171 (h$^-$ ura1) [a derivative strain of ATCC 24969 (C.R. Lab. Carlsberg Ser. Physiol. 24: 381 - 480, (1950)] was transformed with plasmids pFYM201, pFYM225 and pFYM225G4 obtained in Examples 1, 2 and 3, respectively by the method of Beach [D. Beach et al.: Nature 290, 140 - 142 (1981)] and ura$^+$ transformant was obtained. The frequencies of the transformations are described in Table 1.

- 8 -

Table  1

| Plasmid | 1 μg of DNA, $\underline{ura}^+$ transformants per $10^8$ spheroplasts |
| --- | --- |
| pFYM201 | $4.4 \times 10^3$ |
| pFYM225 | $3.9 \times 10^3$ |
| pFYM225G4 | 60 |
| - | 0 |

Among 60 $\underline{ura}^+$ transformants of pFYM225G4, 20 strains were resistant to 100 μg/ml G418 which is one of the gentamicins.

## Example 5

$\underline{Schizosaccharomyces}$ $\underline{pombe}$ JY171 (FERM BP-335) was transformed with plasmid pFYM225G4 in the coexistence of plasmid pFYM2 [Mol. Gen. Genet., $\underline{182}$, 426 - 429 (1981)] as in Example 4.  The results are shown in Table 2.

Table  2

| Plasmid | 1 μg of DNA, $\underline{ura}^+$ transformants per $10^8$ spheroplasts | 1 μg of DNA, $\underline{ura}^+$ and G418 resistant transformants per $10^8$ spheroplasts |
| --- | --- | --- |
| pFYM225G4 | 60 | 20 |
| pFYM225G4 + pFYM2 | $1.0 \times 10^4$ | $8 \times 10^3$ |
| - | 0 | 0 |

Table 2 shows that the coexistence of pFYM2 and pFYM225G4 elevates significantly the frequency of transformation (cotransformation).

WHAT IS CLAIMED IS:

1. A plasmid vector which is a chimera plasmid constructed with a chromosomal DNA fragment of a microorganism belonging to the genus <u>Schizosaccharomyces</u> and a plasmid of a microorganism belonging to the genus <u>Escherichia</u> and is autonomously replicable in both microorganisms belonging to the genera <u>Schizosaccharomyces</u> and <u>Escherichia</u>.

2. The plasmid vector according to claim 1, wherein the microorganism belonging to the genus <u>Schizosaccharomyces</u> is <u>Schizosaccharomyces</u> <u>pombe</u>.

3. The plasmid vector according to claim 1, wherein the chromosomal DNA fragment is a DNA complementing <u>ura1</u> mutation of a microorganism of the genus <u>Schizosaccharomyces</u> or <u>PyrB</u> mutation of a microorganism of <u>Escherichia</u> <u>coli</u>.

4. The plasmid vector according to claim 1, wherein the plasmid of the microorganism belonging to the genus <u>Escherichia</u> is a plasmid of <u>Escherichia</u> <u>coli</u>.

5. The plasmid vector according to claim 4, wherein the plasmid is selected from pBR322, pBR325, pBR327, pBR328, pMB9, pCRl, RSF2124, pBR313, pACYC177 and pACYC184.

6. The plasmid vector according to claim 1, which is characterized in that the plasmid is pFYM201 or pFYM225.

7. A derivative of the plasmid vector, wherein a gene responsible for resistance to an antibiotic other than ampicillin is incorporated in the plasmid vector of claim 1.

8.    The derivative according to claim 7, wherein the gene responsible for resistance to an antibiotic is a gene coding for an enzyme inactivating gentamycin antibiotic G-418.

9.    Plasmid vector pFYM225G4.

0107170

- 12 -

-1μ-

Fig. 1

pFYMZ
9.0kb

pFYM201
6.1kb

pFYM225
4.6kb

Fig. 2

pFYM225G4

6.3kb

0107170

European Patent Office

**EUROPEAN SEARCH REPORT**

Application number

| | DOCUMENTS CONSIDERED TO BE RELEVANT | | EP 83110379.1 |
|---|---|---|---|

| Category | Citation of document with indication, where appropriate, of relevant passages | Relevant to claim | CLASSIFICATION OF THE APPLICATION (Int. Cl. ³) |
|---|---|---|---|
| A,D | NATURE, vol. 375, no. 5676, September 14, 1978 (New York, USA)<br><br>J.D. BEGGS "Transformation of yeast by a replicating hybrid plasmid" pages 104-109<br><br>-- | 1,4 | C 12 N 15/00//<br>C 12 R 1/85<br>C 12 R 1/19 |
| A,D | MOLECULAR & GENERAL GENETICS, vol. 182, no. 3, 1981, (Springer-Verlag, BRD)<br><br>M. YAMAMOTO et al. "Cloning of Gene from the Fission Yeast S. pombe which Complements E. coli pyr B, the Gene of Aspartate Transcarbamylase" pages 426-429<br><br>-- | 1-5 | |
| A | GENE, vol. 1 (1977) (Elsevier/North Holland-Amsterdam; NL)<br><br>C.H. DUNCAN et al. "Transformation of Bacillus subtilis and Escherichia coli by a Hybrid Plasmid p CD1" pages 153-167<br><br>---- | 1,4 | **TECHNICAL FIELDS SEARCHED (Int. Cl. ³)**<br><br>C 12 N |

. The present search report has been drawn up for all claims

| Place of search | Date of completion of the search | Examiner |
|---|---|---|
| VIENNA | 30-12-1983 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82